(19) Europäisches Patentamt
European Patent Office
Office européen des brevets

(11) **EP 1 897 936 A1**

(12) **DEMANDE DE BREVET EUROPEEN**

(43) Date de publication:
**12.03.2008 Bulletin 2008/11**

(51) Int Cl.:
***C12N 5/00*** *(2006.01)* ***C23C 16/24*** *(2006.01)*

(21) Numéro de dépôt: **07115688.9**

(22) Date de dépôt: **05.09.2007**

(84) Etats contractants désignés:
**AT BE BG CH CY CZ DE DK EE ES FI FR GB GR HU IE IS IT LI LT LU LV MC MT NL PL PT RO SE SI SK TR**
Etats d'extension désignés:
**AL BA HR MK YU**

(30) Priorité: **07.09.2006 FR 0653607**

(71) Demandeur: **COMMISSARIAT A L'ENERGIE ATOMIQUE**
**75015 Paris (FR)**

(72) Inventeurs:
• **Borella, Mathias**
  **38000, GRENOBLE (FR)**
• **Plissionier, Marc**
  **38320, EYBENS (FR)**
• **Rouviere, Emmanuelle**
  **38120, SAINT-EGREVE (FR)**
• **Gaillard, Frédéric**
  **38500, VOIRON (FR)**

(74) Mandataire: **Poulin, Gérard et al**
  **Brevatome**
  **3, rue du Docteur Lancereaux**
  **75008 Paris (FR)**

(54) **Substrat de culture pourvu d'un revêtement en silicone oxydée**

(57) Substrat de culture solide comprenant sur au moins l'une de ses surfaces un revêtement en un matériau choisi parmi les silicones oxydées, ledit revêtement comprenant une nanostructuration et/ou une nanorugosité de surface, et une nanoporosité de surface.

Procédé pour cultiver, faire croître des biomolécules vivantes dans lequel on met en contact le substrat avec lesdites biomolécules vivantes et un milieu de culture, croissance.

EP 1 897 936 A1

**Description**

**DOMAINE TECHNIQUE**

La présente invention a trait à un substrat de culture pourvu d'un revêtement en silicone oxydée.

De manière plus précise, la présente invention concerne un substrat de culture comprenant sur au moins l'une de ses surfaces un revêtement en un matériau choisi parmi les silicones oxydées.

Le domaine technique de l'invention peut, de manière générale, être défini comme celui des substrats de culture, c'est-à-dire des substrats mécaniques solides conçus pour favoriser la fixation, l'attachement, la croissance et la prolifération des « biomolécules vivantes » telles que celles constituant les cellules et les tissus vivants comme les nerfs.

Pour les recherches en biologie, microbiologie, médecine et pharmacologie, la culture des cellules et de tissus vivants sur des supports mécaniques est très largement employée.

Pour que les résultats expérimentaux des analyses effectuées sur ces cultures soient pertinents et sans équivoque, il est nécessaire d'adapter au mieux l'environnement de culture de manière à respecter les conditions physiologiques nécessaires aux biomolécules vivantes.

Indépendamment de la présence ou non d'un sérum de culture, le support mécanique utilisé pour la culture est de toute première importance pour un très grand nombre de cellules et de tissus vivants comme cela est indiqué dans le document de Pool and Rappaport, Exp. Cell. Res., 20/465-510, 1960.

Ces substrats doivent offrir une parfaite biocompatibilité, être non toxiques, reproductibles et permettre l'adhésion et la prolifération des cellules de manière à constituer un support de culture viable.

Ainsi, il est aujourd'hui couramment utilisé en biologie, des ustensiles de diverses géométries fabriqués par exemple en polystyrène (PS), en polycarbonate (PC), en polyéthylène tétraphtalate (PET)ou même en verre au silicate.

Les substrats polymères présentent une très bonne biocompatibilité mais une énergie de surface très faible. Pour améliorer leurs performances en termes d'adhésion cellulaire, il est très courant de modifier leur surface par un traitement plasma de décharge par exemple.

Cependant, les substrats décrits ci-dessus se révèlent souvent très inadaptés pour des cultures sensibles (i.e. difficiles) ayant des besoins physiologiques plus complexes, plus particuliers, comme par exemple les cellules primaires, les cellules hépatiques, les cellules épithéliales, les neurones, les bactéries, etc.

L'emploi de substrats standard avec des biomolécules difficiles à cultiver rend la prolifération très lente, l'expression des fonctions peu marquée et la différentiation particulièrement difficile.

Pour résoudre ce problème technique spécifique, un certain nombre de solutions ont déjà été proposées.

Il a été proposé, dans le document US-A-4 243 692, d'augmenter la surface spécifique de contact du support, substrat mécanique de culture par l'enduction d'un substrat plan d'hétéropolycondensats d'oxyde de silicium afin d'offrir un milieu favorisant l'accrochage des organismes et leur développement en un ensemble cohérent. Ce type de procédé reste complexe, onéreux et peu reproductible. Il peut de plus, laisser sur le substrat des produits toxiques issus de la réaction de condensation.

Toujours dans l'idée d'augmenter la surface spécifique de contact et le nombre de point d'ancrage, il a été proposé, dans le document US-A-2005/0095695, de réaliser des cultures cellulaires sur des structures de type empilement de nanofibres de polymères. Ce procédé est efficace mais complexe à mettre en oeuvre et ne garantit aucunement d'être efficace pour la culture de cellules sensibles.

Une solution très intéressante a été proposée dans la demande WO-A-9712966, qui consiste au dépôt par voie liquide de particules de silice nanométriques. Ce procédé présente l'avantage de pouvoir recouvrir une grande variété de géométries ainsi que de permettre un dépôt sur des zones localisées bien spécifiques. Cependant, dans le procédé de ce document, on met en oeuvre des nanopoudres de silice pure qui n'est pas toujours le meilleur substrat pour certaines cellules car il cause un stress important, et l'ajout de composants organiques ne peut se faire que par l'incorporation de particules dans la phase liquide. Enfin, les substrats les plus efficaces et reproductibles sont obtenus par enduction centrifuge « spin coating », ce qui limite beaucoup les géométries de substrats à revêtir.

De plus, tous les procédés cités ci-dessus ne permettent pas de s'affranchir de l'ajout dans le milieu de culture de substances chimiques ayant pour objectif de favoriser l'adhésion et l'expression de fonctions. Bien que couramment utilisés, ces produits ont une action néfaste sur les biomolécules et cause un stress et une mortalité importants, ce qui ne facilite pas toujours la différentiation.

Un substrat permettant de s'affranchir de l'utilisation de ces produits serait une avancée conséquente pour la culture de cellules sensibles.

Il existe au regard de ce qui précède un besoin pour un substrat de culture, pour l'adhésion, la fixation, l'expression de biomolécules vivantes en vue notamment de la culture de cellules et de tissus vivants qui soit parfaitement biocompatible, non toxique, qui permette une excellente adhésion, prolifération, différentiation des cellules et tissus ainsi qu'une excellente expression de fonctions, sans qu'il soit nécessaire d'ajouter dans le milieu de culture des composés promoteurs favorisant l'adhésion, la prolifération, la différentiation ainsi que l'expression de fonctions.

Il existe en outre un besoin pour un tel substrat de culture qui puisse être fabriqué en grand nombre par un procédé simple, rapide, fiable, peu coûteux et reproductible en ce qui concerne la qualité et la nature des substrats obtenus.

Il existe en particulier un besoin pour des substrats de culture qui permettent, tout en étant facilement utilisables et faciles à fabriquer en grand nombre, d'obtenir d'excellents résultats en matière d'adhésion, de prolifération, d'expression des fonctions, et de différentiation lors de la culture de biomolécules, cellules, tissus sensibles, c'est-à-dire difficiles à cultiver, qui nécessitent des ancrages importants, dont l'étude nécessite l'expression de fonctions particulières et dont la différentiation est donc rendue difficile.

Le but de la présente invention est de fournir un substrat de culture qui réponde entre autres aux besoins énumérés ci-dessus.

Le but de la présente invention est encore de fournir un substrat de culture qui ne présente pas les inconvénients, défauts, limitations et désavantages des substrats de l'art antérieur, qui résolve les problèmes posés par les substrats de l'art antérieur et qui réponde à l'ensemble des exigences et critères pour un tel substrat.

Ce but et d'autres encore, sont atteints, conformément à l'invention, par un substrat de culture solide comprenant sur au moins l'une de ses surfaces un revêtement en un matériau choisi parmi les silicones oxydées, ledit revêtement comprenant une nanostructuration et/ou une nanorugosité de surface, et une nanoporosité de surface.

Le substrat de culture selon l'invention est défini par la combinaison de trois caractéristiques essentielles, à savoir : le choix d'une silicone oxydée pour le matériau qui constitue le revêtement, la nanostructuration et/ou la nanorugosité de surface du revêtement, et enfin la nanoporosité de surface du revêtement.

Un tel substrat de culture n'est ni décrit, ni suggéré dans les documents de l'art antérieur.

Le substrat de culture selon l'invention ne présente pas les inconvénients, défauts, limitations et désavantages des substrats de l'art antérieur, il répond aux besoins énumérés plus haut, il satisfait aux critères, exigences pour ce type de substrat, et enfin il apporte une solution aux problèmes posés par les substrats de culture de l'art antérieur.

La réunion, la combinaison, l'association des trois caractéristiques citées plus haut, c'est-à-dire la nanostructuration et/ou la nanorugosité de surface, la nanoporosité de surface, et la conformation de la silicone oxydée (ces caractéristiques, paramètres définissant de préférence un domaine précis) permet, de manière surprenante, d'obtenir d'excellentes performances en matière de culture cellulaire notamment quant à l'adhésion, la prolifération, la différentiation et l'expression de fonctions et cela même sans ajout dans le milieu de culture de promoteurs, ou de substances chimiques favorisant par exemple l'adhésion et l'expression tels que le Dimethyl sulfoxyde (DMSO).

Le substrat de culture de l'invention est notamment particulièrement adapté aux cellules « sensibles », délicates, dont la culture est difficile, qui nécessitent des ancrages importants, dont l'étude nécessite l'expression de fonctions particulières et dont la différentiation était difficile sur les substrats de culture de l'art antérieur.

Le substrat selon l'invention défini par l'association des trois caractéristiques cités ci-dessus est parfaitement adapté à la culture de biomolécules vivantes de manière générale, et donne d'excellents résultats dans le cadre de cette culture mais de manière étonnante, il donne aussi d'excellents résultats, performances pour la culture des cellules primaires, des cellules hépatiques, des neurones, et de toutes les cellules sensibles et délicates. Ainsi, il a pu être observé dans tous les cas une prolifération rapide, et l'expression de fonctions, de préférence sans incorporation de promoteurs, et avec une différentiation très élevée.

L'invention va maintenant être décrite de manière détaillée dans la description qui suit, faite à titre illustratif et non limitatif en référence aux dessins joints, dans lesquels :

- la figure 1 est un graphique tridimensionnel où sont portées le pourcentage de porosité (axe des « Z »), la rugosité Ra (nm), et le %β/(%α+%β) et où a été représenté le domaine de définition préféré du revêtement du substrat selon l'invention.

- les figures 2 et 3 sont des microphotographies prises après deux jours de culture de cellules CHO sur respectivement le substrat 1 et le substrat 2 définis dans l'exemple 2 ci-après.

Le matériau du revêtement du substrat selon l'invention est une silicone oxydée comprenant généralement des groupes choisis parmi les groupes $SiO_2(C, H)_2(\alpha)$, $SiO_3(C,H)(\beta)$ et $SiO_4(\gamma)$.

Selon l'invention, au moins l'un de ces trois groupes est présent, et de préférence les trois.

Mais le matériau du revêtement du substrat selon l'invention peut ne pas être exclusivement constitué à partir des 3 groupes α, β et γ ci-dessus. Il peut comprendre en outre un ou plusieurs autres groupes différents des groupes α, β et γ afin d'assurer un total de 100% tels que les groupes (ω) $SiO (C,H)_3$.

La notation utilisée ci-dessus est claire pour l'homme du métier dans ce domaine de la technique et couramment utilisée. On peut aussi décrire les groupes (α) comme des groupes $SiO_2(R_1)(R_2)$, les groupes (β) comme des groupes $SiO_3(R_1)$, les groupes (γ) comme des groupes $SiO_4$ et les éventuels groupes (ω) comme des groupes $SiO(R_1)(R_2)(R_3)$ ou $R_1$ $R_2$ et $R_3$ identiques ou différents sont des groupements hydrocarbonés tels que des groupements alkyle linéaire ou ramifiées de 1 à 10C comme les groupes méthyle, éthyle, n-propyle, i-propyle, n-butyle.

Les proportions des divers groupes (α), (β) et (γ) sont ajustées notamment en fonction des besoins de l'application et de la culture à réaliser, c'est-à-dire en fonction de la biomolécule à cultiver.

De préférence, les proportions suivantes sont observées : 15 à 35% de groupes (α), 30 à 80% de groupes (β) et 5 à 50% de groupes (γ). En d'autres termes, la silicone oxydée comprend de préférence 15 à 35% de groupes (α), 30 à 80% de groupes (β) et 5 à 50% de groupes (γ), de préférence encore la silicone oxydée est constituée par 15 à 35% de groupes (α), 30 à 80% de groupes (β) et 5 à 50% de groupes (γ).

Il semblerait que dans le cas de telles proportions le « stress » des biomolécules cultivées soit réduit.

En outre, le revêtement du substrat selon l'invention peut être pourvu de fonctions supplémentaires (c'est-à-dire de fonctions ne faisant pas partie initialement du revêtement en silicone oxydée) choisies en fonction de la culture à réaliser, du type de biomolécules à cultiver. Ces fonctions supplémentaires répondent aux besoins physiologiques particuliers de chaque biomolécule et permettent d'améliorer encore les performances du substrat de culture selon l'invention et d'adapter celui-ci exactement à la culture à réaliser.

Ces fonctions supplémentaires peuvent être généralement choisies parmi les groupes hydroxyles (OH), les groupes carboxyles (COOH), les groupes méthyle, et les éléments bioactifs tels que Na et Ca.

Le terme « élément bioactif » est un terme connu par l'homme du métier et couramment utilisé dans ce domaine de la technique.

Ce terme désigne un élément, par exemple d'une particule ou d'une molécule, qui exerce un effet sur une biomolécule par exemple d'une cellule, d'un tissu, ou d'un nerf, etc.

Les effets de l'élément bioactif peuvent être de natures très différentes : adhésion, non-adhésion, croissance, nutrition, différenciation, thérapeutique, électrique, anti-oxydant, etc...

Na et Ca sont des exemples parmi des centaines d'éléments pouvant revêtir un caractère bioactif. Ils ont été choisis pour leur facilité d'intégration au sein même du revêtement de l'invention lors de son élaboration.

La deuxième caractéristique essentielle du substrat de culture selon l'invention est le fait que le revêtement dont il est pourvu comprend une nanostructuration et/ou une nanorugosité de surface. Cette nanostructuration et/ou nanorugosité de surface peut être obtenue soit par une structuration préalable du substrat de base, soit de manière plus avantageuse par le procédé de synthèse du matériau de revêtement.

On entend par « et/ou » dans les termes « nanostructuration et/ou nanorugosité » le fait que le revêtement peut présenter uniquement une nanostructuration géométrique, ou bien uniquement une nanorugosité, ou bien peut présenter une nanorugosité sur une nanostructuration géométrique.

La troisième caractéristique essentielle du substrat selon l'invention est le fait que le revêtement dont il est pourvu présente une nanoporosité de surface qui peut être obtenue par le procédé de synthèse du revêtement ou par post-traitement, impliquant par exemple l'utilisation d'un porogène lors de la synthèse qui est retiré ultérieurement avec génération de pores.

La nanostructuration et/ou rugosité de surface (il s'agit là de la rugosité, y compris de la rugosité sur une structuration) est caractérisée par une rugosité $R_a$ comprise généralement entre 2 nm et 80 nm.

$R_a$ est la rugosité moyenne selon la norme DIN 4768.

Elle est définie par la moyenne arithmétique de toutes les valeurs de rugosité R sur la longueur d'évaluation 1

(i.e. amplitude du profil moyen).

$$R_a = \frac{1}{l} \int_0^l |z| . dx$$

Par nanostructuration on entend généralement que le revêtement comprend des reliefs et/ou des creux.

Ces reliefs et/ou ces creux peuvent notamment se présenter sous l'une ou plusieurs des formes suivantes : tranchées, rainures, gorges, arêtes, cannelures, trous, cavités, plots, pointes, aspérités, bossages, gauffrages, vallées, pics, canaux.

Cette nanostructuration peut en particulier se présenter sous la forme de pics et de vallées avec une hauteur h des pics généralement comprise entre 4 et 160 nm, une largeur 1 des pics comprise entre 2 et 200 nm, par exemple 20 et 200 nm, et un rapport largeur pic/ largeur vallée allant de 0,2 à 2.

La nanoporosité de surface du matériau du revêtement est généralement définie par une porosité supérieure ou égale à 5%, de préférence de 5 à 80%. De façon générale, la porosité est adaptée à la biomolécule à cultiver.

En outre, cette nanoporosité inclut de préférence des pores ouverts d'une taille (diamètre) n'excédant pas généralement 25 nm dans une proportion de 5 à 80% du volume poreux du revêtement.

Il est à noter que le revêtement n'est généralement pas entièrement poreux dans toute son épaisseur et que seule une couche plus ou moins épaisse superficielle est concernée par les pores.

Un matériau particulièrement préféré qui présente des performances particulièrement excellentes pour la culture cellulaire notamment quant à l'adhésion, la prolifération, la différentiation, l'expression des fonctions est défini par des plages particulières de la porosité (en %), de $R_a$ (nm), et du rapport %β/(%α+%γ), et qui sont les suivantes :

- porosité : de 5 à 80%
- $R_a$ (nm) : de 2 à 80 nm
- Rapport %β/(%α+%γ) : de 0,5 à 4.

Les 3 groupes α, β et γ ne font pas toujours 100% au total et il est donc tout à fait possible d'obtenir le rapport qui est mentionné ci-dessus.

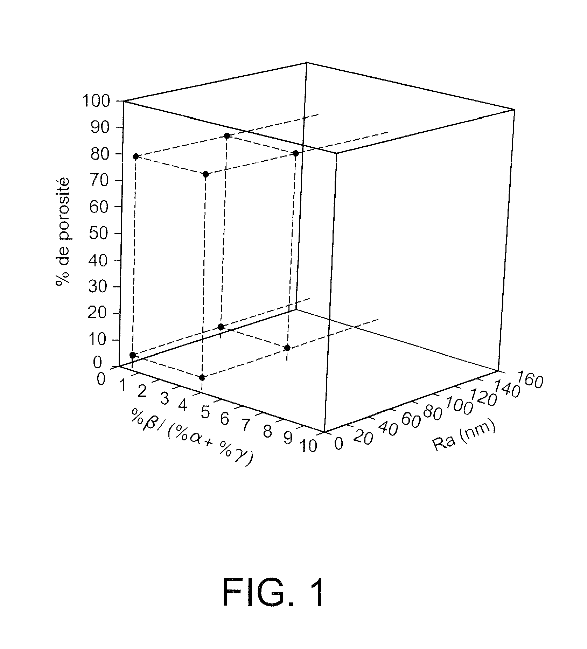

Le matériau défini par les plages particulières de chacun de ces trois paramètres peut être représenté par le domaine indiqué sur la figure 1. L'épaisseur dudit revêtement est généralement de 10 à 100 nm de préférence de 20 à 50 nm, de préférence de 30 à 40 nm. Des épaisseurs de 10, 15 ou 25 nm peuvent être utilisées.

Une épaisseur faible de matériau telle que définie ci-dessus est suffisante mais il est néanmoins tout à fait possible d'employer des épaisseurs plus importantes, à savoir par exemple un micron, ou plusieurs microns.

L'avantage d'une épaisseur faible réside dans le fait que le matériau reste très largement transparent dans le visible et présente une fluorescence induite très faible facilitant ainsi les analyses optiques.

Le substrat (support, substrat de base à distinguer du substrat de culture qui inclut, le support, substrat de base et le revêtement) peut être réalisé en tout autre matériau solide adéquat.

Il peut s'agir de tout matériau solide connu de l'homme du métier tel que par exemple les matériaux supports utilisés pour la fabrication des microsystèmes d'analyse et des biopuces. Le matériau du substrat peut être organique ou inorganique, minéral. Il peut être en un matériau choisi parmi le verre, la silice, les polycarbonates, les polyméthacrylates de méthyle (PMMA),les polystyrènes, les polyéthylènes téréphtalates ; le substrat peut être aussi un substrat composite comprenant plusieurs matériaux différents choisis par exemple parmi ceux énumérés ci-dessus.

Le substrat qui est recouvert en totalité ou en partie par le revêtement tel que décrit ci-dessus peut avoir une forme quelconque, il peut s'agir en particulier de tout instrument, ustensile, dispositif utilisé traditionnellement par l'homme du métier pour la culture des molécules biologiques vivantes, des cellules et des tissus.

Le substrat selon l'invention peut être préparé selon un premier mode de réalisation par un procédé dans lequel on dépose par PECVD, en une seule étape, à partir d'un précurseur organosilicié mélangé avec de l'oxygène, un revêtement en un matériau choisi parmi les silicones oxydées tel que décrit plus haut sur un substrat.

Dans ce mode de réalisation du procédé, les trois caractéristiques du matériau selon l'invention sont obtenues simultanément, en une seule étape.

Le substrat présente une nanostructuration et/ou une nanorugosité qui se retrouve dans la couche déposée.

Le précurseur est choisi parmi les organosiliciés tels que l'hexaméthyldisiloxane, l'octaméthylcyclotétrasiloxane, le décaméthyltétrasiloxane.

L'homme du métier ajustera les proportions du mélange de précurseur et d'oxygène, et les autres paramètres de la décharge tels que la puissance dissipée, la pression de travail, les débits, de manière à obtenir une silicone oxydée ayant la composition recherchée et les proportions de groupements (α), (β), (γ) désirées.

La pression de travail est un paramètre important pour l'obtention de la porosité souhaitée.

Le substrat selon l'invention peut être préparé selon un second mode de réalisation par un procédé dans lequel on réalise les étapes successives suivantes :

- on dépose par PECVD à partir d'un précuseur organosilicié mélangé avec un gaz neutre un revêtement très peu réticulé et très organique,
- on traite le revêtement ainsi obtenu avec un plasma de gaz neutre ou réducteur dans les conditions favorisant la génération d'espèces gazeuses au sein du matériau afin de générer la nanoporosité et la nanorugosité.

Les termes « très peu réticulé et « très organique » sont connus par l'homme du métier et sont des termes couramment utilisés et connus dans ce domaine de la technique.

Les termes « très réticulé » ou « peu réticulé » (en anglais « high or low reticulation ») ont une signification bien connue et signifient que le matériau est d'autant moins réticulé qu'il présente un maximum de groupements chimiques présents dans le précurseur.

Le terme « très organique » signifie que les groupements présentant des carbones sont majoritaires ce qui correspond par exemple à une forte présence de groupes α et/ou ω.

Le précurseur mis en oeuvre dans la première étape est choisi en fonction de son aptitude à permettre au matériau solide de revêtement de former des pores et de la rugosité a posteriori lors de la deuxième étape. C'est-à-dire que la molécule de précurseur doit permettre de former un dépôt solide tout en conservant au maximum sa partie organique.

Ce précurseur peut être choisi parmi les organosiliciés tels que l'octaméthylcyclotétrasiloxane, l'hexaméthylcyclotrisiloxane, et le décaméthyltétrasiloxane.

Le précurseur est dilué dans un gaz neutre tel que l'argon ou l'hélium.

Les conditions de dépôt lors de la première étape sont telles qu'elles permettent le dépôt d'un revêtement très peu réticulé et très organique afin de permettre au traitement de la deuxième étape d'être efficace. En d'autres termes, il faut préserver la partie organique constituée par exemple par les groupements hydrocarbonés, par exemple alkyle tel que méthyle des précurseurs.

Le revêtement obtenu à l'issue de la première étape pourra comprendre ainsi, généralement, au minimum 60% de groupements (α), environ 20% de groupements (ω), au maximum 20% de groupements (β) et 0% de groupements (γ).

En fonction de la nature du revêtement obtenu à l'issue de la première étape, on ajustera le traitement de la deuxième étape. Celui-ci consiste à favoriser la formation d'espèces gazeuses au sein même du matériau de revêtement à partir de ses constituants organiques, c'est-à-dire des groupes hydrocarbonés tels que les groupes alkyle comme les (-CH$_3$) sans oxydation de ceux-ci, car sinon la formation de porosité et de rugosité n'a pas lieu.

Il est donc préférable d'employer un plasma de gaz neutre comme l'argon ou l'hélium ou réducteur comme l'hydrogène avec une puissance dissipée et un temps de traitement adaptés au matériau initial par exemple respectivement de quelques dixièmes de W/cm$^2$ et de l'ordre de la minute et sous une pression par exemple comprise entre 10 et 10 Pa.

En réglant les paramètres du traitement au plasma de la deuxième étape, on obtient un revêtement ayant la composition souhaitée définie par les proportions des groupements ($\alpha$), ($\beta$) et ($\gamma$), la nanoporosité de surface et les nanorugosités de surface voulues. De préférence, on fait bien entendu en sorte que la composition du revêtement, la rugosité et la porosité se trouvent dans le domaine préféré indiqué plus haut et représenté sur la figure 1.

La composition du revêtement à savoir les proportions des divers groupements $\alpha$, $\beta$, $\gamma$, la nanoporosité de surface et la nanorugosité de surface peuvent être ajustées en fonction des molécules biologiques, cellules, tissus que l'on souhaite cultiver.

L'invention concerne également un procédé pour cultiver, faire croître des biomolécules vivantes dans lequel on met en contact le substrat tel que décrit plus haut avec lesdites biomolécules et un milieu de culture, croissance.

L'invention va maintenant être décrit en référence aux exemples suivants, donnés à titre illutratif et non limitatif.

**EXEMPLES**

**Exemple 1**

Dans cet exemple, on décrit la fabrication d'un substrat de culture selon l'invention, particulièrement adapté aux besoins physiologiques d'une lignée cellulaire d'hépatocytes hepaRG.

Le substrat utilisé est une lame de verre standard de microscopie optique qui présente une rugosité de surface de R$_a$ de 9 nm.

Dans une première étape, on dépose sur ce substrat un matériau de type silicone très peu réticulé par dépôt chimique en phase vapeur assisté par plasma (« PECVD »).

Le précurseur utilisé dans cet exemple est le décaméthyltétrasiloxane (C$_{10}$H$_{30}$O$_3$Si$_4$).

Ce précurseur est dilué dans de l'hélium dans une proportion de 20%.

Les conditions du dépôt sont les suivantes : 0,2 W/cm$^2$, 25 Pa, 75 secondes.

On obtient à l'issue de cette première étape un revêtement d'une épaisseur de 100 nm sur le substrat en verre.

Le matériau déposé comprend au minimum 60% de groupements ($\alpha$), environ 20% de groupements SiO(C,H)$_3$, au maximum 20% de groupements ($\beta$) et 0% de groupements ($\gamma$).

Le matériau solide ainsi déposé a une conformation très peu réticulée et très organique, c'est-à-dire que cette conformation très proche de celle de la molécule de précuseur avec la préservation des groupes organiques de cette dernière. Les termes « (très peu) réticulée » et « très organique » ont été définis en détail plus haut.

Dans une deuxième étape, on effectue le traitement du revêtement déposé lors de la première étape à l'aide d'un plasma d'hélium avec une puissance dissipée de 0,5 W/cm$^2$ et une durée de traitement de 60 secondes sous une pression de 50 Pa. De telles conditions favorisent la formation d'espèces gazeuses telles que CH$_4$, C$_2$H$_2$, C$_2$H$_4$ au sein même du matériau à partir de ses constituants organiques (essentiellement les groupes CH$_3$) sans oxydation de ceux-ci, il en résulte la formation d'une porosité et d'une rugosité de surface du matériau déposé lors de la première étape.

Le traitement plasma de la seconde étape conduit à un revêtement en un matériau comprenant 25% de groupements ($\alpha$), 65% de groupements ($\beta$) et 10% de groupements ($\gamma$) à +/-10%.

Ce traitement a en outre permis d'obtenir un revêtement comprenant une rugosité R$_a$ de 3 nm environ, qui se superpose à la rugosité du substrat en verre, et une porosité de surface d'environ 10%, sur 10 à 15 nm d'épaisseur.

La rugosité R$_a$ du substrat a été déterminée par microscopie à force atomique et la porosité a été mesurée par ellipsométrie spectroscopique.

Ce substrat s'avère particulièrement adapté à la cellule d'une lignée cellulaire d'hépatocytes hepa RG.

Avec le substrat selon l'invention préparé dans cet exemple, les hépatocytes ont vu leur temps de génération, c'est-à-dire le temps pour doubler le nombre de cellules, divisé par deux par rapport à un substrat de culture standard en polystyrène.

De plus, les cellules montrent un stress minimum inférieur à tous les substrats de cultures de l'art antérieur tels que le verre et le polystyrène de culture utilisés à titre de comparaison.

Enfin, la différentiation des cellules est d'une importante exceptionnelle sans addition de promoteur.

Le stress et la différentiation (qui est le comportement individuel par rapport au comportement du groupe) sont des critères et notions bien connus en biologie qui sont appréciés généralement de manière qualitative.

**Exemple 2**

Dans cet exemple, on compare des cultures réalisées sur deux substrats différents dont l'un (le substrat 2) est conforme à l'invention.

Le substrat 1 est caractérisé par la présence d'une silicone oxydée comprenant 10% de (α), 25% de (β) et 65% de (γ), une rugosité Ra de 10 nm et une porosité nulle.

Le substrat 2 est caractérisé par la présence d'une silicone oxydée comprenant 30% de (α), 55% de (β) et 15% de (γ), une rugosité Ra de 18 nm et une porosité de surface d'environ 20% sur 20 à 25 nm d'épaisseur.

Plus précisément, on compare dans cet exemple la croissance de cellules de type CHO (Chinese Hamster Ovary) sur ces deux substrats. Le mode opératoire est le suivant :

Une lignée de cellule CHO (Chinese Hamster Ovarian Cell), cellules adhérentes a été cultivée dans une boîte de pétri jusqu'à confluence. Les cellules ont été recueillies par trypsination comme suit.

Le milieu de culture est retiré. La culture est nettoyée par deux lavages de 2-3 ml de PBS. On ajoute ensuite environ 1 ml de trypsine par 50 cm$^2$ de surface à trypsiner. On place la culture à 37°C pendant 1 minute. La trypsine est retirée délicatement par pipetage. On remet la boîte à 37°C pendant 3 minutes. Les cellules sont récupérées dans du milieu de culture (DMEM + antibiotique) par agitation et « fluschage ». Elles sont re-suspendues dans un volume au moins 3 fois plus important puis l'ensemble est « vortexé ».

Des lames, comprenant, formant le substrat 1 ou le substrat 2, ont été placées par 3 dans des boîtes de culture carrées de 120 cm$^2$ de surface. Chaque boîte a été ensemencée avec 50 ml de suspension de culture à raison de 6,6 6 x 10$^4$ cellules/ml (soit une répartition a priori de 55 cellules/cm$^2$ dans la boîte).

Les deux substrats 1 et 2 permettent d'obtenir pour les cellules CHO un temps de génération comparable de l'ordre de 20 h.

Le temps de génération est défini par

$$Tg = \frac{(T - T_0) \times \ln 2}{\ln N - \ln N_0} \; , \; Tg \text{ temps de génération.}$$

T-T$_0$ temps de comptage. N nombre de cellules à T. N$_0$, nombre de cellules à T$_0$.

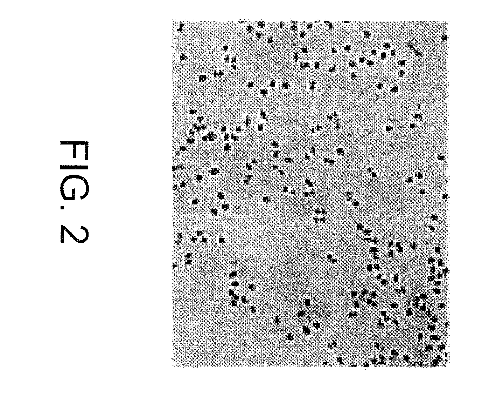

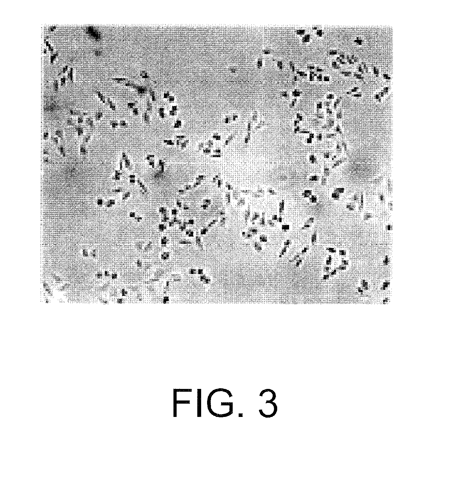

Cependant, l'importance de l'étalement des cellules après 2 jours de culture atteste du stress minimum procuré par le substrat 2 conforme à l'invention et de sa meilleure adéquation aux besoins physiologiques des cellules (voir figures 2 et 3).

## Revendications

1. Substrat de culture solide comprenant sur au moins l'une de ses surfaces un revêtement en un matériau choisi parmi les silicones oxydées, ledit revêtement comprenant une nanostructuration et/ou une nanorugosité de surface, et une nanoporosité de surface.

2. Substrat selon la revendication 1, dans lequel la nanostructuration et/ou nanorugosité, est **caractérisée par** une rugosité R$_a$ selon la norme DIN 4768 comprise entre 2 nm et 80 nm.

3. Substrat selon l'une quelconque des revendications précédentes, dans lequel la nanostructuration de surface comprend des reliefs et/ou des creux.

4. Substrat selon la revendication 3, dans lequel la nanostructuration de surface du revêtement se présente sous une ou plusieurs des formes suivantes : tranchées, rainures, gorges, arêtes, cannelures, trous, cavités, plots, pointes, aspérités, bossages, gauffrages, vallées, pics, canaux.

5. Substrat selon la revendication 4, dans lequel la nanostructuration et/ou nanorugosité de surface du revêtement se présente sous la forme de pics et de vallées avec une hauteur h des pics comprise entre 4 et 160 nm, une largeur 1 des pics comprise entre 2 et 200 nm, et un rapport largeur pic/largeur vallée allant de 0,2 à 2.

6. Substrat selon l'une quelconque des revendications précédentes, dans lequel la nanoporosité du matériau est supérieure ou égale à 5%, de préférence de 5 à 80%.

7. Substrat selon l'une quelconque des revendications précédentes, dans lequel la nanoporosité comprend des pores ouverts d'une taille ne dépassant pas 25 nm dans une proportion de 5 à 80% du volume poreux du revêtement.

8. Substrat selon l'une quelconque des revendications précédentes, dans lequel le matériau du revêtement comprend des groupes choisis parmi les groupes SiO$_2$(C,H)$_2$(α), SiO$_3$(C,H)(β) et SiO$_4$(γ).

9. Substrat selon la revendication 8, dans lequel le matériau du revêtement comprend en outre un ou plusieurs autres groupes différents des groupes (α), (β) et (γ) tels que des groupes SiO(C,H)$_3$ (ω).

10. Substrat selon la revendication 8, dans lequel le matériau du revêtement comprend de 15 à 35% de groupes (α), de 30 à 80% de groupes (β) et de 5 à 50% de groupes (γ).

11. Substrat selon l'une quelconque des revendications précédentes dans lequel le revêtement du sustrat en outre est pourvu de fonctions supplémentaires choisies parmi les groupes hydroxyle, carboxyle, les groupes méthyle, et les éléments bioactifs tels que Na et Ca.

**12.** Substrat selon l'une quelconque des revendications précédentes, dans lequel l'épaisseur dudit revêtement est de 10 à 100 nm, de préférence de 20 à 50 nm, de préférence encore de 30 à 40 nm.

**13.** Procédé de préparation du substrat de culture selon l'une quelconque des revendications 1 à 12, dans lequel on dépose, sur un substrat, par dépôt chimique en phase vapeur assisté par plasma (PECVD), en une seule étape, à partir d'un précurseur organosilicié mélangé avec de l'oxygène, un revêtement en un matériau choisi parmi les silicones oxydées.

**14.** Procédé selon la revendication 13, dans lequel ledit précurseur organosilicié est choisi parmi l'hexaméthyldisiloxane, l'octaméthylcyclotétrasiloxane, et le décaméthyltétrasiloxane.

**15.** Procédé de préparation du substrat de culture selon l'une quelconque des revendications 1 à 12, dans lequel on réalise les étapes successives suivantes :

    - on dépose par PECVD à partir d'un précuseur organosilicié mélangé avec un gaz neutre un revêtement très peu réticulé et très organique,
    - on traite le revêtement ainsi obtenu avec un plasma de gaz neutre ou réducteur dans des conditions favorisant la génération d'espèces gazeuses au sein du matériau afin de générer la nanoporosité et la nanorugosité.

**16.** Procédé pour cultiver, faire croître des biomolécules vivantes dans lequel on met en contact le substrat selon l'une quelconque des revendications 1 à 12 avec lesdites biomolécules vivantes et un milieu de culture, croissance.

FIG. 1

FIG. 2

FIG. 3

Office européen
des brevets

**RAPPORT DE RECHERCHE EUROPEENNE**

Numéro de la demande

EP 07 11 5688

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X | FISSELL W H ET AL: "Differentiated growth of human renal tubule cells on thin-film and nanostructured materials" ASAIO JOURNAL 2006 UNITED STATES, vol. 52, no. 3, juin 2006 (2006-06), pages 221-227, XP009080611 ISSN: 1058-2916 * le document en entier * | 1-12,16 | INV. C12N5/00 C23C16/24 |
| Y | DEN BRABER E T ET AL: "Quantitative analysis of fibroblast morphology on microgrooved surfaces with various groove and ridge dimensions" BIOMATERIALS, ELSEVIER SCIENCE PUBLISHERS BV., BARKING, GB, vol. 17, no. 21, novembre 1996 (1996-11), pages 2037-2044, XP004033026 ISSN: 0142-9612 paragraphe "Materials and methods" * abrégé * | 1-12 | |
| Y | SORDEL T ET AL: "Hourglass SiO2 coating increases the performance of planar patch-clamp" JOURNAL OF BIOTECHNOLOGY 20 AUG 2006 NETHERLANDS, vol. 125, no. 1, 20 août 2006 (2006-08-20), pages 142-154, XP002424759 ISSN: 0168-1656 * abrégé; tableau 1 * | 1-12 | DOMAINES TECHNIQUES RECHERCHES (IPC)  C12N |

-/--

Le présent rapport a été établi pour toutes les revendications

| Lieu de la recherche | Date d'achèvement de la recherche | Examinateur |
|---|---|---|
| Munich | 24 septembre 2007 | LOUBRADOU-BOURGES, N |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
.......................................................
& : membre de la même famille, document correspondant

EPO FORM 1503 03.82 (P04C02)

**EP 1 897 936 A1**

**Office européen des brevets**

**RAPPORT DE RECHERCHE EUROPEENNE**

Numéro de la demande

EP 07 11 5688

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| Y | MEYLE J ET AL: "Variation in contact guidance by human cells on a microstructured surface" J BIOMED MATER RES; JOURNAL OF BIOMEDICAL MATERIALS RESEARCH JAN 1995 JOHN WILEY & SONS INC, NEW YORK, NY, USA, vol. 29, no. 1, janvier 1995 (1995-01), pages 81-88, XP009080568 paragraphe "Characterization of substratum surface" * page 83 * ----- | 1-12 | |
| A | FLEMMING R G ET AL: "EFFECTS OF SYNTHETIC MICRO-AND NANO-STRUCTURED SURFACES ON CELL BEHAVIOR" BIOMATERIALS, ELSEVIER SCIENCE PUBLISHERS BV., BARKING, GB, vol. 20, mars 1999 (1999-03), pages 573-588, XP002926697 ISSN: 0142-9612 * le document en entier * ----- | 1-16 | DOMAINES TECHNIQUES RECHERCHES (IPC) |
| D,A | WO 97/12966 A (CORNING INC [US]) 10 avril 1997 (1997-04-10) * revendications 1-22 * ----- | 1-16 | |

Le présent rapport a été établi pour toutes les revendications

| Lieu de la recherche | Date d'achèvement de la recherche | Examinateur |
|---|---|---|
| Munich | 24 septembre 2007 | LOUBRADOU-BOURGES, N |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)

13

**EP 1 897 936 A1**

## ANNEXE AU RAPPORT DE RECHERCHE EUROPEENNE
## RELATIF A LA DEMANDE DE BREVET EUROPEEN NO.

EP 07 11 5688

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

24-09-2007

| Document brevet cité au rapport de recherche | Date de publication | Membre(s) de la famille de brevet(s) | Date de publication |
|---|---|---|---|
| WO 9712966 A | 10-04-1997 | AU 7514296 A<br>EP 0853664 A1<br>JP 11513254 T | 28-04-1997<br>22-07-1998<br>16-11-1999 |

EPO FORM P0460

Pour tout renseignement concernant cette annexe : voir Journal Officiel de l'Office européen des brevets, No.12/82

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 4243692 A
- US 20050095695 A
- WO 9712966 A

**Littérature non-brevet citée dans la description**

- **POOL ; RAPPAPORT.** *Exp. Cell. Res,* 1960, vol. 20, 465-510